# EUROPÄISCHE PATENTANMELDUNG

(11) **EP 1 247 868 A2**
(43) Veröffentlichungstag der Anmeldung: **09.10.2002**
(21) Anmeldenummer: 02001400.7
(22) Anmeldetag: 19.01.2002
(51) Int. Cl.: C12P 13/02, C12N 1/21, A23K 1/16

(54) **Verfahren zur fermentativen Herstellung von D-Pantothensäure und/oder deren Salzen**

(30) Priorität: 03.04.2001 DE 10116519; 23.10.2001 US 983167
(71) Anmelder: Degussa AG, 40474 Düsseldorf (DE)
(72) Erfinder: Dusch, Nicole, Dr., 33824 Werther (DE); Marx, Achim, Dr., 33790 Halle (DE); Pfefferle, Walter, Dr., 33790 Halle (DE); Thierbach, Georg, Dr., 33613 Bielefeld (DE)

(57) **Zusammenfassung**

Gegenstand der Erfindung ist ein Verfahren zur fermentativen Herstellung von D-Pantothensäure und/oder deren Salzen oder diese enthaltende Futtermitteladditive durch Fermentation von coryneformen Bakterien, insbesondere solchen, die bereits D-Pantothensäure produzieren, dadurch gekennzeichnet, dass man das (die) für die glyA-Gen kodierende(n) Nukleotidsequenz(en) in den coryneformen Bakterien verstärkt, insbesondere überexprimiert.

## Beschreibung

Diese Erfindung betrifft ein Verfahren zur fermentativen Herstellung von D-Pantothensäure und/oder deren Salzen oder diese enthaltende Mischungen unter Verwendung coryneformer Bakterien, in denen mindestens das glyA-Gen verstärkt wird.

### Stand der Technik

Pantothensäure wird weltweit in einer Größenordnung von mehreren tausend Tonnen pro Jahr produziert. Es wird unter anderem in der Humanmedizin, in der pharmazeutischen Industrie und in der Lebensmittelindustrie verwendet. Ein großer Teil der produzierten Pantothensäure wird für die Ernährung von Nutztieren wie Geflügel und Schweine verwendet. Der Bedarf steigt.

Pantothensäure kann durch chemische Synthese oder biotechnisch durch Fermentation geeigneter Mikroorganismen in geeigneten Nährlösungen hergestellt werden. Bei der chemischen Synthese ist das DL-Pantolacton eine wichtige Vorstufe. Es wird in einem mehrstufigen Verfahren aus Formaldehyd, Isobutylaldehyd und Cyanid hergestellt, in weiteren Verfahrensschritten das racemische Gemisch aufgetrennt, das D-Pantolacton mit β-Alanin kondensiert und so D-Pantothensäure erhalten.

Die typische Handelsform ist das Calcium-Salz der D-Pantothensäure. Das Calcium-Salz des racemischen Gemisches der D,L-Pantothensäure ist ebenfalls gebräuchlich.

Der Vorteil der fermentativen Herstellung durch Mikroorganismen liegt in der direkten Bildung der gewünschten stereoisomeren Form, nämlich der D-Form, die frei von L-Pantothensäure ist.

Verfahren zur Herstellung von D-Pantothensäure mit Hilfe von Corynebacterium glutamicum sind in der Literatur nur ansatzweise bekannt. So berichten Sahm und Eggeling (Applied and Environmental Microbiology 65(5), 1973-1979, (1999)) über den Einfluss der Überexpression der Gene panB und panC und Dusch et al. (Applied and Environmental Microbiology 65(4), 1530-1539, (1999)) über den Einfluss des Gens panD auf die Bildung der D-Pantothensäure.

### Aufgabe der Erfindung

Die Erfinder haben sich zur Aufgabe gestellt, neue Maßnahmen zur verbesserten fermentativen Herstellung von D-Pantothensäure und/oder deren Salzen bzw. diese enthaltende Tierfuttermittel-Additive bereitzustellen.

### Beschreibung der Erfindung

Wenn im Folgenden D-Pantothensäure oder Pantothensäure oder Pantothenat erwähnt werden, sind damit nicht nur die freien Säuren, sondern auch die Salze der D-Pantothensäure wie z.B. das Calcium-, Natrium-, Ammonium- oder Kaliumsalz gemeint.

Gegenstand der Erfindung ist ein Verfahren zu Herstellung von D-Pantothensäure und/oder deren Salzen oder diese enthaltende Futtermitteladditive durch Fermentation von coryneformen Bakterien, insbesondere solchen, die bereits D-Pantothensäure produzieren, bei dem man
a) das (die) in den Bakterien die für das endogene glyA-Gen kodierende(n) Nukleotidsequenz(en) verstärkt, insbesondere überexprimiert, unter Bedingungen die für die Produktion der Serinhydroxymethyltransferase geeignet sind,
b) die D-Pantothensäure und/oder deren Salze im Medium oder in den Zellen der Mikroorganismen anreichert, und
c) die gewünschten Produkte nach Abschluss der Fermentation isoliert, wobei man die Biomasse und/oder gegebenenfalls weitere Bestandteile der Fermentationsbrühe in einer Menge von ≥ 0 bis 100 % abtrennt,
wobei die Bakterien D-Pantothensäure produzieren.

Gegenstand der Erfindung ist ebenso ein Verfahren, bei dem nach Abschluss der Fermentation die Biomasse teilweise oder insgesamt in der Fermentationsbrühe verbleibt und die so erhaltene Brühe gegebenenfalls nach dem Aufkonzentrieren zu einer festen D-Pantothensäure und/oder deren Salze enthaltenden Mischung, die auch weitere Bestandteile der Fermentationsbrühe enthält, verarbeitet wird.

Der Begriff "Verstärkung" beschreibt in diesem Zusammenhang die Erhöhung der intrazellulären Aktivität eines oder mehrerer Enzyme bzw. Proteine in einem Mikroorganismus, die durch die entsprechende DNA kodiert werden, indem man beispielsweise die Kopienzahl des Gens bzw. der Gene erhöht, einen starken Promotor oder ein Gen oder Allel verwendet, das für ein entsprechendes Enzym bzw. Protein mit einer hohen Aktivität kodiert und gegebenenfalls diese Maßnahmen kombiniert.

Durch die Maßnahmen der Verstärkung, insbesondere Überexpression, wird die Aktivität oder Konzentration des entsprechenden Proteins im allgemeinen um mindestens 10%, 25%, 50%, 75%, 100%, 150%, 200%, 300%, 400% oder 500%, maximal bis 1000% oder 2000% bezogen auf die des Wildtyp-Proteins beziehungsweise der Aktivität oder Konzentration des Proteins im Ausgangs-Mikroorganismus erhöht.

Die Mikroorganismen, die Gegenstand der vorliegenden Erfindung sind, können D-Pantothensäure aus Glucose, Saccharose, Lactose, Fructose, Maltose, Melasse, Stärke, Cellulose oder aus Glycerin und Ethanol herstellen. Es handelt sich um Vertreter coryneformer Bakterien, insbesondere der Gattung Corynebacterium. Bei der Gattung Corynebacterium ist insbesondere die Art Corynebacterium glutamicum (C. glutamicum) zu nennen, die in der Fachwelt für ihre Fähigkeit bekannt ist, L-Aminosäuren zu produzieren.

Geeignete Stämme der Gattung Corynebacterium, insbesondere der Art Corynebacterium glutamicum, sind beispielsweise die bekannten Wildtypstämme
Corynebacterium glutamicum ATCC13032
Corynebacterium acetoglutamicum ATCC15806
Corynebacterium acetoacidophilum ATCC13870
Corynebacterium thermoaminogenes FERM BP-1539
Brevibacterium flavum ATCC14067
Brevibacterium lactofermentum ATCC13869 und
Brevibacterium divaricatum ATCC14020
und daraus hergestellte, D-Pantothensäure produzierende Mutanten wie beispielsweise
Corynebacterium glutamicum ATCC13032ΔilvA/pEC7panBC
Corynebacterium glutamicum ATCC13032/pND-D2

Es wurde gefunden, dass coryneforme Bakterien nach Überexpression des für das Serinhydroxymethyltransferase kodierenden glyA-Gens in verbesserter Weise Pantothensäure produzieren.

Das glyA-Gen codiert für das Enzym Serinhydroxymethyltransferase (EC 2.1.2.1). Die Nukleotidsequenz des glyA-Gens wurde in der japanischen Offenlegungsschrift JP-A-08107788 beschrieben. Das in der angegebenen Textstelle beschriebene glyA-Gen kann erfindungsgemäß verwendet werden. Weiterhin können Allele des glyA-Gens verwendet werden, die sich aus der Degeneriertheit des genetischen Codes oder durch funktionsneutrale Sinnmutationen ("sense mutations") ergeben, die zu keiner grundsätzlichen Veränderung der Aktivität des Proteins führen. Derartige Mutationen werden unter anderem auch als neutrale Substitutionen bezeichnet.

Zur Erzielung einer Verstärkung (z.B. Überexpression) erhöht man z.B. die Kopienzahl der entsprechenden Gene oder mutiert die Promotor- und Regulationsregion oder die Ribosomenbindungsstelle, die sich stromaufwärts des Strukturgens befindet. In gleicher Weise wirken Expressionskassetten, die stromaufwärts des Strukturgens eingebaut werden. Durch induzierbare Promotoren ist es zusätzlich möglich, die Expression im Verlaufe der fermentativen Pantothensäure-Bildung zu steigern. Durch Maßnahmen zur Verlängerung der Lebensdauer der m-RNA wird ebenfalls die Expression verbessert. Weiterhin wird durch Verhinderung des Abbaus des Enzymproteins ebenfalls die Enzymaktivität verstärkt. Die Gene oder Genkonstrukte liegen dabei entweder in Plasmiden mit unterschiedlicher Kopienzahl vor oder sind im Chromosom integriert und amplifiziert. Alternativ kann weiterhin eine Überexpression der betreffenden Gene durch Veränderung der Medienzusammensetzung und Kulturführung erreicht werden.

Anleitungen hierzu findet der Fachmann unter anderem bei Martin et al. (Bio/Technology 5, 137-146 (1987)), bei Guerrero et al. (Gene 138, 35-41 (1994)), Tsuchiya und Morinaga (Bio/Technology 6, 428-430 (1988)), bei Eikmanns et al. (Gene 102, 93-98 (1991)), in EP 0 472 869, im US Patent 4,601,893, bei Schwarzer und Pühler (Bio/Technology 9, 84-87 (1991), bei Reinscheid et al. (Applied and Environmental Microbiology 60, 126-132 (1994)), bei LaBarre et al. (Journal of Bacteriology 175, 1001-1007 (1993)), in der Patentanmeldung WO 96/15246, bei Malumbres et al. (Gene 134, 15-24 (1993)), in der japanischen Offenlegungsschrift JP-A-10-229891, bei Jensen und Hammer (Biotechnology and Bioengineering 58, 191-195 (1998)) und in bekannten Lehrbüchern der Genetik und Molekularbiologie.

Beispielsweise kann das glyA-Gen mit Hilfe von Plasmiden überexprimiert werden.

Als Plasmide eignen sich solche, die in coryneformen Bakterien repliziert werden. Zahlreiche bekannte Plasmidvektoren wie z.B. pZ1 (Menkel et al., Applied and Environmental Microbiology (1989), 64: 549-554), pEKEx1 (Eikmanns et al., Gene 102:93-98 (1991)), oder pHS2-1 (Sonnen et al., Gene 107:69-74 (1991)) beruhen auf den kryptischen Plasmiden pHM1519, pBL1 oder pGA1. Andere Plasmidvektoren wie z.B. solche, die auf pCG4 (US-A 4,489,160), oder pNG2 (Serwold-Davis et al., FEMS Microbiology Letters 66, 119-124 (1990)), oder pAG1 (US-A 5,158,891) beruhen, können in gleicher Weise verwendet werden.

Weiterhin eignen sich solche Plasmidvektoren mit Hilfe derer man das Verfahren der Genamplifikation durch Integration in das Chromosom anwenden kann, so wie es beispielsweise von Reinscheid et al. (Applied and Environmental Microbiology 60, 126-132 (1994)) zur Duplikation bzw. Amplifikation des hom-thrB-Operons beschrieben wurde.

Bei dieser Methode wird das vollständige Gen in einen Plasmidvektor kloniert, der in einem Wirt (typischerweise E. coli), nicht aber in C. glutamicum replizieren kann. Als Vektoren kommen beispielsweise pSUP301 (Simon et al., Bio/Technology 1, 784-791 (1983)), pK18mob oder pK19mob (Schäfer et al., Gene 145, 69-73 (1994)), pGEM-T (Promega Corporation, Madison, WI, USA), pCR2.1-TOPO (Shuman (1994). Journal of Biological Chemistry 269:32678-84; US-A 5,487,993), pCR®Blunt (Firma Invitrogen, Groningen, Niederlande; Bernard et al., Journal of Molecular Biology, 234: 534-541 (1993)) oder pEM1 (Schrumpf et al, 1991, Journal of Bacteriology 173:4510-4516) in Frage. Der Plasmidvektor, der das zu amplifizierende Gen enthält, wird anschließend durch Konjugation oder Transformation in den gewünschten Stamm von C. glutamicum überführt. Die Methode der Konjugation ist beispielsweise bei Schäfer et al. (Applied and Environmental Microbiology 60, 756-759 (1994)) beschrieben. Methoden zur Transformation sind beispielsweise bei Thierbach et al. (Applied Microbiology and Biotechnology 29, 356-362 (1988)), Dunican und Shivnan (Bio/Technology 7, 1067-1070 (1989)) und Tauch et al. (FEMS Microbiological Letters 123, 343-347 (1994)) beschrieben. Nach homologer Rekombination mittels eines "cross over"-Ereignisses enthält der resultierende Stamm mindestens zwei Kopien des betreffenden Gens.

Zusätzlich kann es für die Produktion von Pantothensäure vorteilhaft sein, neben dem endogenen glyA-Gen ein oder mehrere weitere für Enzyme des Serin-Biosyntheseweges, des Keto-Isovaleriansäure-Biosyntheseweges, des Asparaginsäure-Biosyntheseweges, des Pantothensäure-Biosyntheseweges, der Glycolyse oder der Anaplerotik kodierende Gene zu verstärken.

So kann zusätzlich eines oder mehrere der endogenen Gene ausgewählt aus der Gruppe:
- das für die 3-Phosphoglycerat-Dehydrogenase kodierende serA-Gen oder ein für eine "feed back" resistente 3-Phosphoglycerat-Dehydrogenase kodierendes serA-Allel (US-A-6,037,154),
- das für die Phosphoserin-Transaminase kodierende serC-Gen, (WO01/00843)
- das für die Phosphoserin-Phosphatase kodierende serB-Gen, (WO01/00843)
- das für die Acetohydroxysäure-Synthase kodierende ilvBN-Operon (Journal of Bacteriology 175, 5595-5603 (1993)),
- das für die Dihydroxysäure-Dehydratase kodierende ilvD-Gen (JP-A-1996089249),
- das für die Acetohydroxysäure-Isomeroreduktase kodierende ilvC-Gen (EP-A-1001027),
- das für die Ketopantoat-Hydroxymethyltransferase kodierende panB-Gen (EP-A-1006189),
- das für die Pantothenat-Synthetase kodierende panC-Gen (EP-A-1006189),
- das für die Aspartat-Decarboxylase kodierende panD-Gen (EP-A-1006192),
- das für die Phosphofructokinase kodierende pfkA-Gen (DE: 19956133.8 und DE: 10030702.7) und
- das für die Pyruvat-Carboxylase kodierende pyc-Gen (DE-A-19831609 und DE: 19943055.1)
verstärkt, insbesondere überexprimiert werden.

Weiterhin kann es für die Produktion von Pantothensäure vorteilhaft sein, zusätzlich zur Verstärkung des für die Serin-Hydroxymethyltransferase kodierenden glyA-Gens eines oder mehrerer der Gene ausgewählt aus der Gruppe:
- das für die Phosphoenolpyruvat-Carboxykinase (PEP-Carboxykinase) kodierende pck-Gen (DE: 19950409.1) und
- das für die Pyruvat-Oxidase kodierende poxB-Gen (DE: 19951975.7 und DE: 10048604.5)
abzuschwächen, insbesondere auszuschalten oder die Expression zu verringern.

Der Begriff "Abschwächung" beschreibt in diesem Zusammenhang die Verringerung oder Ausschaltung der intrazellulären Aktivität eines oder mehrerer Enzyme (Proteine) in einem Mikroorganismus, die durch die entsprechende DNA kodiert werden, indem man beispielsweise einen schwachen Promotor verwendet oder ein Gen bzw. Allel verwendet, das für ein entsprechendes Enzym (Protein) mit einer niedrigen Aktivität kodiert bzw. das entsprechende Gen oder Enzym (Protein) inaktiviert und gegebenenfalls diese Maßnahmen kombiniert.

Durch die Maßnahmen der Abschwächung einschließlich der Verringerung der Expression wird die Aktivität oder Konzentration des entsprechenden Proteins im allgemeinen auf 0 bis 75%, 0 bis 50%, 0 bis 25%, 0 bis 10% oder 0 bis 5% der Aktivität oder Konzentration des Wildtyp-Proteins, beziehungsweise der Aktivität oder Konzentration des Proteins im Ausgangs-Mikroorganismus, herabgesenkt.

Weiterhin kann es für die Produktion von D-Pantothensäure vorteilhaft sein neben der Überexpression des glyA-Gens unerwünschte Nebenreaktionen auszuschalten (Nakayama: "Breeding of Amino Acid Producing Microorganisms", in:
Overproduction of Microbial Products, Krumphanzl, Sikyta, Vanek (eds.), Academic Press, London, UK, 1982). In dem erfindungsgemäßen Verfahren können Bakterien eingesetzt werden in denen die Stoffwechselwege zumindest teilweise ausgeschaltet sind, die die Bildung der D-Pantothensäures verringern.

Die erfindungsgemäß hergestellten Mikroorganismen können im batch - Verfahren (Satzkultivierung), im fed batch (Zulaufverfahren) oder im repeated fed batch - Verfahren (repetitives Zulaufverfahren) kultiviert werden. Eine Zusammenfassung über bekannte Kultivierungsmethoden ist im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) beschrieben.

Das zu verwendende Kulturmedium muss in geeigneter Weise den Ansprüchen der jeweiligen Stämme genügen. Beschreibungen von Kulturmedien verschiedener Mikroorganismen sind im Handbuch "Manual of Methods for General Bacteriology" der American Society for Bacteriology (Washington D.C., USA, 1981) enthalten. Als Kohlenstoffquelle können Zucker und Kohlehydrate wie z.B. Glucose, Saccharose, Lactose, Fructose, Maltose, Melasse, Stärke und Cellulose, Öle und Fette wie z.B. Sojaöl, Sonnenblumenöl, Erdnussöl und Kokosfett, Fettsäuren wie z.B. Palmitinsäure, Stearinsäure und Linolsäure, Alkohole wie z.B. Glycerin und Ethanol und organische Säuren wie z.B. Essigsäure verwendet werden. Diese Stoffe können einzeln oder als Mischung verwendet werden.

Als Stickstoffquelle können organische Stickstoff haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden.

Als Phosphorquelle können Phosphorsäure, Kaliumdihydrogenphosphat oder Dikaliumhydrogenphosphat oder die entsprechenden Natrium haltigen Salze verwendet werden. Das Kulturmedium muß weiterhin Salze von Metallen enthalten wie z.B. Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden. Dem Kulturmedium können überdies Vorstufen der D-Pantothensäure wie Aspartat, β-Alanin, Ketoisovalerat, Ketopantoinsäure oder Pantoinsäure und gegebenenfalls deren Salze zugesetzt werden. Die genannten Einsatzstoffe können zur Kultur in Form eines einmaligen Ansatzes hinzugegeben oder in geeigneter Weise während der Kultivierung zugefüttert werden.

Zur pH-Kontrolle der Kultur werden basische Verbindungen wie Natriumhydroxid, Kaliumhydroxid, Ammoniak bzw. Ammoniakwasser oder saure Verbindungen wie Phosphorsäure oder Schwefelsäure in geeigneter Weise eingesetzt.

Es ist gleichfalls möglich, zur Darstellung der Erdalkalisalze der Pantothensäure, insbesondere des Calciumsalzes, während der Fermentation die Suspension oder Lösung einer Erdalkali-haltigen anorganischen Verbindung wie beispielsweise Kalziumhydroxid oder einer organischen Verbindung wie dem Erdalkalisalz einer organischen Säure beispielsweise Calciumacetat, kontinuierlich oder diskontinuierlich zu versetzen. Auf diese Weise wird das zur Darstellung des gewünschten Erdalkalisalzes der D-Pantothensäure nötige Kation direkt in der gewünschten Menge, im allgemeinen in einem Verhältnis von 0,8 bis 1,2 zu 1, bezogen auf die Pantothensäure, bevorzugt in stöchiometrischen Mengen, in die Fermentationsbrühe eingetragen.

Zur Kontrolle der Schaumentwicklung können Antischaummittel wie z.B. Fettsäurepolyglykolester eingesetzt werden. Zur Aufrechterhaltung der Stabilität von Plasmiden können dem Medium geeignete selektiv wirkende Stoffe z.B. Antibiotika hinzugefügt werden. Um aerobe Bedingungen aufrechtzuerhalten, werden Sauerstoff oder Sauerstoff haltige Gasmischungen wie z.B. Luft in die Kultur eingetragen. Die Temperatur der Kultur liegt normalerweise bei 25°C bis 45°C und vorzugsweise bei 30°C bis 40°C. Die Kultur wird solange fortgesetzt bis sich ein Maximum an D-Pantothensäure gebildet hat. Dieses Ziel wird normalerweise innerhalb von 10 Stunden bis 160 Stunden erreicht.

Die in der Fermentationsbrühe enthaltene D-Pantothensäure bzw. die entsprechenden Salze der D-Pantothensäure kann anschließend nach dem Stand der Technik isoliert und gereinigt werden.

Es ist ebenso möglich, die D-Pantothensäure und/oder deren Salze enthaltenden Fermentationsbrühen vorzugsweise zunächst durch bekannte Separationsmethoden wie zum Beispiel der Zentrifugation, der Filtration, dem Dekantieren oder einer Kombination hieraus vollständig oder zum Teil (≥ 0 bis 100 %) von der Biomasse zu befreien. Es ist jedoch auch möglich, die Biomasse gänzlich in der Fermentationsbrühe zu belassen. Im allgemeinen wird die Suspension oder Lösung vorzugsweise aufkonzentriert und beispielsweise mit Hilfe eines Sprühtrockners oder einer Gefriertrocknungsanlage zu einem Pulver aufgearbeitet. Anschließend wird dieses Pulver im allgemeinen durch geeignete Kompaktier- oder Granulier-Verfahren z. B. auch Aufbaugranulation in ein gröberkörniges, gut rieselfähiges, lagerbares und weitgehend staubfreies Produkt mit einer Korngrößenverteilung von 20 bis 2000 µm, insbesondere 100 bis 1400 µm, überführt. Vorteilhaft bei der Granulation oder Kompaktierung ist der Einsatz von üblichen organischen oder anorganischen Hilfsstoffen, beziehungsweise Trägern wie Stärke, Gelatine, Cellulosederivaten oder ähnlichen Stoffen, wie sie üblicherweise in der Lebensmittel- oder Futterverarbeitung als Binde-, Gelier-, oder Verdickungsmittel Verwendung finden, oder von weiteren Stoffen wie zum Beispiel Kieselsäuren, Silikaten oder Stearaten.

Alternativ kann das Fermentationsprodukt mit oder ohne weitere der üblichen Bestandteile der Fermentationsbrühe auf einen in der Futtermittelverarbeitung bekannten und üblichen organischen oder anorganischen Trägerstoff wie zum Beispiel Kieselsäuren, Silikate, Schrote, Kleien, Mehle, Stärken Zucker oder andere aufgezogen und/oder mit üblichen Verdickungs- oder Bindemitteln stabilisiert werden. Anwendungsbeispiele und Verfahren hierzu sind in der Literatur (Die Mühle + Mischfuttertechnik 132 (1995) 49, Seite 817) beschrieben.

Gegebenenfalls wird auf einer geeigneten Verfahrensstufe D-Pantothensäure oder das gewünschte Salz der D-Pantothensäure oder eine diese Verbindungen enthaltende Zubereitung hinzugefügt, um den gewünschten Gehalt an Pantothensäure oder dem gewünschten Salz zu erzielen bzw. einzustellen.

Der gewünschte Gehalt liegt im allgemeinen im Bereich von 20 bis 80 Gew.-% (Trockenmasse).

Die Konzentration an Pantothensäure kann mit bekannten chemischen (Velisek; Chromatographic Science 60, 515-560 (1992)) oder mikrobiologischen Verfahren wie z.B. dem Lactobacillus plantarum Test (DIFCO MANUAL, 10^{th} Edition, S. 1100-1102; Michigan, USA) bestimmt werden.

Die vorliegende Erfindung wird im folgenden anhand von Ausführungsbeispielen näher erläutert.

Zu diesem Zweck wurden Versuche mit dem Isoleucinbedürftigen Stamm ATCC13032ΔilvA, dem Plasmid pND-D2 und dem Plasmid pVWEx2glyA durchgeführt.

Eine Reinkultur des Stammes ATCC13032ΔilvA ist am 21. Oktober 1998 als DSM12455 bei der Deutschen Sammlung für Mikroorganismen und Zellkulturen in Braunschweig (Deutschland) gemäss Budapester Vertrag hinterlegt worden. Das panD-Gen haltige Plasmid pND-D2 ist bei Dusch et al. (Applied and Environmental Microbiology 65(4), 1530-1539 (1999)) beschrieben und in Form einer Reinkultur des Stammes Corynebacterium glutamicum ATCC13032/pND-D2 am 05. Oktober 1998 als DSM12438 ebenfalls bei der Deutschen Sammlung für Mikroorganismen und Zellkulturen in Braunschweig (Deutschland) gemäss Budapester Vertrag hinterlegt worden. Das Plasmid pVWEx2glyA ist der EP-A-1106695 beschrieben. Es wurde in Form einer Reinkultur des Escherichia coli Stammes DH5alphamcr/pVWEx2glyA am 3. August 2001 bei der Deutschen Sammlung für Mikroorganismen und Zellkulturen in Braunschweig (Deutschland) gemäss Budapester Vertrag hinterlegt unter der Nummer DSM 14443.

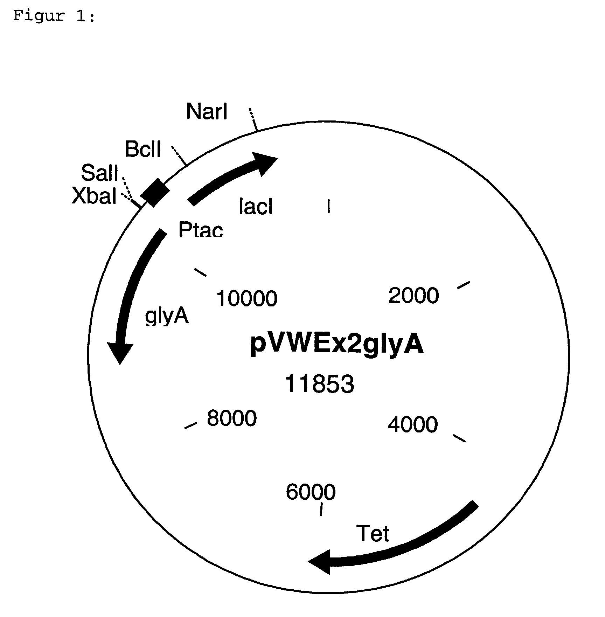

Eine Karte des Plasmids pVWEx2glyA ist in der Figur 1 dargestellt.

### Beispiel 1

### Herstellung des Stammes ATCC13032ΔilvA/pND-D2, pVWEx2glyA

Nach Elektroporation (Tauch et.al., 1994, FEMS Microbiological Letters, 123:343-347) des Plasmids pND-D2 in den C. glutamicum Stamm ATCC13032ΔilvA und anschließender Selektion auf LB-Agar (Lennox, 1955, Virology, 1:190-206), der mit 25 µg/ml Kanamycin supplementiert worden war, wurde der Stamm
ATCC13032ΔilvA/pND-D2 erhalten.

Nach Elektroporation des Plasmids pVWEx2glyA in den C. glutamicum Stamm ATCC13032AilvA/pND-D2 und anschließender Selektion auf LB-Agar, der mit 25 µg/ml Kanamycin und 10 µg/ml Tetracyclin supplementiert worden war, wurde der Stamm ATCC13032ΔilvA/pND-D2, pVWEx2glyA erhalten.

### Beispiel 2

### Herstellung von Pantothensäure

Die Bildung von Pantothenat durch die C. glutamicum Stämme ATCC13032ΔilvA/pND-D2 und ATCC13032ΔilvA/pND-D2, pVWEx2glyA wurde in Medium CGXII (Keilhauer et al., 1993, Journal of Bacteriology, 175:5595-5603; Tabelle 1) geprüft, das mit 25 µg/ml Kanamycin, 2 mM Isoleucin und im Falle des Stammes ATCC13032ΔilvA/pND-D2, pVWEx2glyA mit zusätzlich 10 µg/ml Tetracyclin supplementiert worden war.

Dieses Medium wird im Folgenden als C. glutamicum-Testmedium bezeichnet. Je 50 ml frisch angesetztes C. glutamicum-Testmedium wurden aus einer 16 Stunden alten Vorkultur des gleichen Mediums dergestalt angeimpft, dass die optische Dichte der Kultursuspension (o.D.₅₈₀) bei Inkubationsbeginn 0,1 betrug. Die Kulturen wurden bei 30°C und 130 U/min bebrütet. Nach 5 stündiger Inkubation wurde IPTG (Isopropyl β-D-thiogalactosid) in einer Endkonzentration von 1 mM hinzugefügt. Nach 48 stündiger Inkubation wurde die optische Dichte (o.D.₅₈₀) der Kultur bestimmt und anschließend die Zellen durch 10minütige Zentrifugation bei 5000 g entfernt und der Überstand sterilfiltriert.

Zur Bestimmung der optischen Dichte wurde ein Novaspec II Photometer der Firma Pharmacia (Freiburg, Deutschland) bei einer Messwellenlänge von 580 nm eingesetzt.

Die Quantifizierung des D-Pantothenats im Kulturüberstand erfolgte mittels Lactobacillus plantarum ATCC 8014 nach Angaben des Handbuchs der Firma DIFCO (DIFCO MANUAL, 10^{th} Edition, S. 1100-1102; Michigan, USA). Für die Kalibrierung wurde das Hemicalciumsalz von Pantothenat der Firma Sigma (Deisenhofen, Deutschland) verwendet.

Das Ergebnis ist in Tabelle 2 dargestellt.

**Tabelle 1**

| Substanz | Menge pro Liter | Bemerkung |
|---|---|---|
| (NH₄)₂ SO₂ | 20 g | |
| Harnstoff | 5 g | |
| KH₂PO₄ | 1 g | |
| K₂HPO₄ | 1 g | |
| MgSO₄ * 7 H₂O | 0.25 g | |
| MOPS | 42 g | |
| CaCl₂ | 10 mg | |
| FeSO₄ * 7 H₂O | 10 mg | |
| MnSO₄ * H₂O | 10 mg | |
| ZnSO₄ * 7 H₂O | 1 mg | |
| CuSO₄ | 0.2 mg | |
| NiCl₂ * 6 H₂O | 0.02 mg | |
| Biotin | 0.5 mg | |
| Glukose | 40 g | separat autoklavieren |
| Protocatechusäure | 0.03 mg | sterilfiltrieren |

**Tabelle 2**

| Stamm | Zelldichte oD₅₈₀ | Konzentration (ng/ml) |
|---|---|---|
| ATCC13032ΔilvA/pND-D2 | 14,9 | 30 |
| ATCC13032ΔilvA/pND-D2, pVWEx2glyA | 14,8 | 51 |

### Kurze Beschreibung der Figur:

### Figur 1: Karte des Plasmids pVWEx2glyA

Bei der Angabe der Basenpaarzahlen handelt es sich um Näherungswerte, die im Rahmen der Reproduzierbarkeit von Messungen erhalten werden.

Die verwendeten Abkürzungen und Bezeichnungen haben folgende Bedeutung.
- Tet:: Resistenzgen für Tetracyclin
- lacI:: lacI-Repressor
- Ptac:: tac-Promotor
- glyA:: Serin-Hydroxymethyltransferase Gen aus C. glutamicum

- BclI:: Schnittstelle des Restriktionsenzyms BamHI
- NarI:: Schnittstelle des Restriktionsenzyms NarI
- SalI:: Schnittstelle des Restriktionsenzyms SalI
- XbaI:: Schnittstelle des Restriktionsenzyms XbaI

## Patentansprüche

1. Verfahren zu Herstellung von D-Pantothensäure und/oder deren Salzen oder diese enthaltende Futtermitteladditive durch Fermentation von coryneformen Bakterien, insbesondere solchen, die bereits D-Pantothensäure produzieren, bei dem man
a) das (die) in den Bakterien die für das endogene glyA-Gen kodierende(n) Nukleotidsequenz(en) verstärkt, insbesondere überexprimiert, unter Bedingungen die für die Produktion der Serinhydroxymethyltransferase geeignet sind,
b) die D-Pantothensäure und/oder deren Salze im Medium oder in den Zellen der Mikroorganismen anreichert, und
c) die gewünschten Produkte nach Abschluss der Fermentation isoliert, wobei man die Biomasse und/oder gegebenenfalls weitere Bestandteile der Fermentationsbrühe in einer Menge von ≥ 0 bis 100 % abtrennt,
wobei die Mikroorganismen D-Pantothensäure produzieren.

2. Verfahren gemäß Anspruch 1, **dadurch gekennzeichnet, dass** man die Fermentation in Gegenwart von Erdalkalisalzen durchführt, wobei diese kontinuierlich oder diskontinuierlich in der gewünschten Menge zugeführt werden, und ein Erdalkalisalze der D-Pantothensäure enthaltendes oder daraus bestehendes Produkt gewinnt.

3. Verfahren gemäß Anspruch 1, **dadurch gekennzeichnet, dass** man coryneformen Bakterien der Gattung Corynebacterium einsetzt.

4. Verfahren gemäß Anspruch 3, **dadurch gekennzeichnet, dass** die coryneformen Bakterien der Gattung Corynebacterium aus der Art Corynebacterium glutamicum stammen.

5. Verfahren gemäß Anspruch 1, **dadurch gekennzeichnet, dass** man zusätzlich zu dem endogenen glyA-Gen eines oder mehrere der endogenen Gene, ausgewählt aus der Gruppe:
5.1 das für die 3-Phoshoglycerat-Dehydrogenase kodierende serA-Gen oder ein für eine "feed back" resistente 3-Phoshoglycerat-Dehydrogenase kodierendes serA-Allel,
5.2 das für die Phosphoserin-Transaminase kodierende serC-Gen,
5.3 das für die Phosphoserin-Phosphatase kodierende serB-Gen,
5.4 das für die Acetohydroxysäure-Synthase kodierende ilvBN-Operon,
5.5 das für die Dihydroxysäure-Dehydratase kodierende ilvD-Gen,
5.6 das für die Acetohydroxysäure-Isomeroreduktase kodierende ilvC-Gen,
5.7 das für die Ketopantoat-Hydroxymethyltransferase kodierende panB-Gen,
5.8 , das für die Pantothenat-Synthetase kodierende panC-Gen
5.9 das für die Aspartat-Decarboxylase kodierende panD-Gen,
5.10 das für die Pantothenat-Synthetase kodierende panC-Gen,
5.11 das für die Phosphofructokinase kodierende pfkA-Gen, und
5.12 das für die Pyruvat-Carboxylase kodierende pyc-Gen,
verstärkt, insbesondere überexprimiert.

6. Verfahren gemäß Anspruch 1, **dadurch gekennzeichnet, dass** man Bakterien einsetzt, in denen die Stoffwechselwege zumindest teilweise ausgeschaltet sind, die die Bildung der D-Pantothensäure verringern, insbesondere das für Phosphoenolpyruvat-Carboxykinase (PEP-Carboxykinase) kodierende pck-Gen und/oder das für die Pyruvat-Oxidase kodierende poxB-Gen ausschaltet oder auf niedrigem Niveau exprimiert.

7. Verfahren gemäß Anspruch 1, **dadurch gekennzeichnet, dass** die Aktivität oder Konzentration des glyA-Genprodukts (Proteins) um 10 bis 2000 % bezogen auf die des Wildtypproteins bzw. des Proteins im Ausgangs-Mikroorganismus erhöht ist.

8. Verfahren gemäß Anspruch 1, **dadurch gekennzeichnet, dass** man transformierte Stämme einsetzt, in denen das glyA-Gen in Plasmiden oder im Chromosom integriert vorliegt und verstärkt wird.

9. Ein Vektor zur Expression des glyA-Gens von C. glutamicum, der einen Promoter und die Gensequenz enthält.

10. Coryneforme Bakterien, insbesondere der Gattung Corynebacterium, transformiert mit einem Vektor gemäß Anspruch 9.

11. Coryneforme Bakterien, insbesondere der Gattung Corynebacterium, transformiert mit dem Plasmid pVWEx2glyA, dargestellt in Figur 1.

12. Verfahren zur Herstellung von D-Pantothensäure und/oder deren Salzen durch Fermentation von Mikroorganismen gemäß den Ansprüchen 10 oder 11.

13. Verfahren gemäß Anspruch 1, bei dem man
a) aus einer durch Fermentation gewonnenen D-Pantothensäure und/oder deren Salze enthaltenden Fermentationsbrühe, die Biomasse und/oder gegebenenfalls die Inhaltsstoffe der Fermentationsbrühe in einer Menge von ≥ 0 bis 100 % abtrennt,
b) das so erhaltene Gemisch gegebenenfalls aufkonzentriert, und
c) das die Pantothensäure und/oder das Pantothenat enthaltende Gemisch durch geeignete Maßnahmen in eine rieselfähige Form überführt, und
d) daraus ein rieselfähiges Tierfuttermittel-Additiv mit einer Korngrößenverteilung von 20 bis 2000 µm, insbesondere 100 bis 1400 µm herstellt.

14. Verfahren zur Herstellung von Tierfuttermittel-Additiven gemäß Anspruch 2 mit einem Gehalt an D-Pantothensäure und/oder deren Salzen ausgewählt aus der Gruppe Magnesium- oder Kalziumsalz, bei dem man
a) gegebenenfalls Wasser aus der Fermentationsbrühe (Aufkonzentration) entfernt,
b) die während der Fermentation gebildete Biomasse in einer Menge von ≥ 0 bis 100% abtrennt,
c) gegebenenfalls eine oder mehrere der genannten Verbindungen zu den gemäß a) und b) behandelten Fermentationsbrühen zusetzt, wobei die Menge der zugesetzten Verbindungen so bemessen ist, dass deren Gesamtkonzentration im Tierfuttermittel-Additiv im Bereich von etwa 20 bis 80 Gew.-% (Trockenmasse) liegt, und
d) das Tierfuttermittel-Additiv in der gewünschten Pulver- oder bevorzugt Granulatform herstellt.

15. Verfahren gemäß Anspruch 14, **dadurch gekennzeichnet, dass** man aus der Fermentationsbrühe gegebenenfalls nach Zusatz von D-Pantothensäure und/oder deren Salzen und gegebenenfalls nach Zusatz von organischen oder anorganischen Hilfsstoffen durch
a) Trocknen und Kompaktieren, oder
b) Sprühtrocknen, oder
c) Sprühtrocknen und Granulieren, oder
d) Sprühtrocknen und Aufbaugranulieren,
ein Tierfuttermittel-Additiv in der gewünschten Pulveroder Granulatform gewinnt.
